## Europäisches Patentamt

## European Patent Office

## Office européen des brevets

(11) Veröffentlichungsnummer: **0 179 217**
A2

# (12) EUROPÄISCHE PATENTANMELDUNG

(21) Anmeldenummer: 85110341.6

(22) Anmeldetag: 19.08.85

(51) Int. Cl.⁴: **C 07 D 209/88**

(30) Priorität: 27.08.84 DE 3431416

(43) Veröffentlichungstag der Anmeldung:
30.04.86 Patentblatt 86/18

(84) Benannte Vertragsstaaten:
CH DE FR LI NL

(71) Anmelder: HOECHST AKTIENGESELLSCHAFT
Postfach 80 03 20
D-6230 Frankfurt am Main 80(DE)

(72) Erfinder: Tronich, Wolgang, Dr.
Adolf-Guckes-Weg 5
D-6239 Eppstein/Taunus(DE)

(72) Erfinder: Wykypiel, Werner, Dr.
Egerstrasse 7
D-6054 Rodgau(DE)

(54) Verfahren zur Herstellung von 2-Hydroxy-5,6,7,8-tetrahydrocarbazol, die Alkali- und Erdalkalisalze des 2-Hydroxy-5,6,7,8-tetrahydrocarbazols und deren Verwendung.

(57) 2-Hydroxy-5,6,7,8-tetrahydrocarbazol und dessen Alkali- und Erdalkalisalze werden hergestellt, indem man 2-Hydroxy-cyclohexanon mit m-Aminophenol in wäßrigem Medium oder wäßrig-organischem Medium - wobei das organische Lösemittel ein mit Wasser mischbares, polares organisches Lösemittel ist - bei einer Temperatur zwischen 20 und 100°C und einem pH-Wert zwischen 7,5 und 1 umsetzt, das gebildete 2-Hydroxy-5,6,7,8-tetrahydrocarbazol aus der Reaktionsmischung als Alkali- oder Erdalkalisalz abscheidet und dieses Salz gegebenenfalls mittels einer Mineralsäure in das freie 2-Hydroxy-5,6,7,8-tetrahydrocarbazol überführt.

EP 0 179 217 A2

HOECHST AKTIENGESELLSCHAFT    HOE 84/F 196      Dr.ST

Verfahren zur Herstellung von 2-Hydroxy-5,6,7,8-tetra-
hydrocarbazol, die Alkali- und Erdalkalisalze des
2-Hydroxy-5,6,7,8-tetrahydrocarbazols und deren Verwendung

Die Erfindung liegt auf dem technischen Gebiet der Herstellung von Carbazolverbindungen als Zwischenprodukte für
die Synthese von Farbstoffen.

2-Hydroxy-carbazol dient als wertvolles Zwischenprodukt
zur Herstellung von Farbstoffen. Es kann aus 2-Hydroxy-
5,6,7,8-tetrahydrocarbazol nach im Prinzip bekannten
Methoden der Dehydrierung erhalten werden (s. bspw.
J. Chem. Soc. 1955, 3475-3477). 2-Hydroxy-5,6,7,8-tetrahydrocarbazol selbst läßt sich auf verschiedenem Wege herstellen. So wird im Beispiel 4 der deutschen Patentschrift Nr. 574 840 (s. Friedländer, Fortschritte der
Teerfarbenfabrikation, Springer Verlag, Berlin, Band 19
(1934), S. 820 u. 821) dessen Synthese durch Umsetzung von
m-Hydroxy-phenylhydrazin und Cyclohexanon in wäßrigem,
saurem Medium beschrieben; die technische Durchführung
dieser Verfahrensweise ist jedoch heutzutage uninteressant,
da die Herstellung des m-Hydroxy-phenylhydrazins schwierig
und ökologisch problematisch ist. Auch das in dieser Hinsicht verbesserte Verfahren gemäß den Beispielen 7 und 8
der US-Patentschrift 2 731 474, bei dem die Zwischenisolierung von m-Hydroxy-phenylhydrazin umgangen wird, ist
technisch nicht befriedigend, da hier zunächst als Zwischenprodukt das m-Hydroxy-phenylhydrazon des Cyclohexanons hergestellt wird, das vor dessen Umsetzung zum
Hydroxy-tetrahydrocarbazol erst aus wäßrigem Alkohol zur
Reinigung umkristallisiert wird; bei der anschließenden
Ringschlußreaktion in Eisessig/Salzsäure bilden sich zudem zwei isomere Hydroxy-tetrahydrocarbazole, von denen
das gewünschte 2-Hydroxy-5,6,7,8,-tetrahydrocarbazol erst
durch aufwendige Umkristallisation isoliert werden kann.

Eine andere bekannte Verfahrensweise der Herstellung von 2-Hydroxy-5,6,7,8-tetrahydrocarbazol ist die Umsetzung von m-Aminophenol mit reinem 2-Hydroxy-cyclohexanon in der Schmelze bei einer Temperatur von oberhalb 100°C; aus dieser Schmelze wird das Tetrahydrocarbazol mit Methanol ausgelöst, mit Wasser ausgefällt und durch Sublimation gereinigt (s. Chem. Ber. 92, 2387 u. 2395 (1959)). Das nach dieser Verfahrensweise hergestellte Rohprodukt enthält jedoch gemäß der Analyse nach der Hochdruck-Flüssigkeitschromatographie das gewünschte 2-Hydroxy-5,6,7,8-tetrahydrocarbazol nur zu 60 bis 70 %; es ist braun gefärbt und hat einen breiten Schmelzpunktbereich von 110 bis 150°C. Erst durch Sublimation wird ein reines Produkt vom Schmelzpunkt von 163 bis 164°C erhalten.

Für die Weiterverarbeitung des 2-Hydroxy-5,6,7,8-tetrahydrocarbazols zum 2-Hydroxy-carbazol ist es allerdings wichtig, daß es besonders rein ist, um der erforderlichen katalytischen Dehydrierung unterworfen werden zu können und um zu vermeiden, daß der bei der Dehydrierung verwendete Edelmetallkatalysator, wie z.B. Palladium auf Aktivkohle, infolge vorhandener Verunreinigungen des Ausgangsproduktes unwirksam wird. Die hierfür erforderliche Reinigung durch Sublimation bedeutet allerdings einen technisch erheblichen Aufwand.

Es bestand somit die Aufgabe, reines 2-Hydroxy-5,6,7,8-tetrahydrocarbazol in einem technisch wirtschaftlichen und auch ökologisch akzeptablen Verfahren aufzufinden. Diese Aufgabe wurde mit der vorliegenden Erfindung gelöst.

Die vorliegende Erfindung betrifft somit ein Verfahren zur Herstellung von 2-Hydroxy-5,6,7,8-tetrahydrocarbazol und dessen Alkali- und Erdalkalisalze durch Umsetzung von 2-Hydroxy-cyclohexanon mit m-Aminophenol, das dadurch gekennzeichnet ist, daß man die Umsetzung dieser beiden Verbindungen in wäßrigem Medium bei einem pH-Wert zwischen 7,5 und 1 und einer Temperatur zwischen 20 und 100°C durchführt, das gebildete 2-Hydroxy-5,6,7,8-tetrahydrocarbazol aus der Reaktionsmischung als Alkalisalz oder Erdalkalisalz abscheidet und isoliert und gewünschtenfalls dieses Alkali- oder Erdalkalisalz mittels einer Mineralsäure in das 2-Hydroxy-5,6,7,8-tetrahydrocarbazol überführt.

Die Umsetzung des 2-Hydroxy-cyclohexanons mit dem m-Aminophenol erfolgt in wäßrigem Medium; vorteilhaft ist jedoch die Umsetzung in einem wäßrigen Medium, das ein mit Wasser mischbares, polares organisches Lösemittel enthält, wie bspw. ein Alkanon von 2 bis 5 C-Atomen oder ein Carbonsäureamid einer aliphatischen Carbonsäure von 1 bis 4 C-Atomen, wie Dimethylformamid, oder ein Alkanol von 1 bis 5 C-Atomen oder ein Alkan-polyol von 2 bis 8 C-Atomen, wie Ethylenglykol; als polares, mit Wasser mischbares organisches Lösemittel dient bevorzugt Methanol. In der Regel liegt das Mischungsverhältnis von Wasser zu dem organischen Lösemittel zwischen 10:1 und 1:1 Gewichtsteilen. Beim Einsatz von Methanol als zusätzliches polares Lösemittel hat sich ein Mischungsverhältnis von Wasser zu Methanol zwischen 3:1 und 1:1 Gewichtsteilen bewährt.

Im allgemeinen führt man die Umsetzung zwischen dem 2-Hydroxycyclohexanon und dem m-Aminophenol bei einem pH-Wert zwischen 6 und 2 durch; bevorzugt erfolgt sie zwischen 5,5 und 2, insbesondere zwischen 5 und 3. Der pH-Wert wird durch Zugabe einer Mineralsäure, wie beispielsweise Salzsäure oder Schwefelsäure, oder einer starken bis mittelstarken organischen Säure, wie Essigsäure, Chloressigsäure oder Trifluoressigsäure oder Ameisensäure, eingestellt.
Vorteilhaft ist es weiterhin, die Umsetzung in einem abgeschlossenen Gefäß unter der Atmosphäre eines inerten Gases, wie unter einer Stickstoffatmosphäre, durchzuführen.

Reaktionstemperatur liegt bevorzugt zwischen 40 und . Vorteilhaft beginnt man die Umsetzung der beiden Komponenten im neutralen Medium innerhalb eines pH-Bereiches von 5,5 bis 7, d.h. ohne Zusatz einer Säure, um zunächst die intermediäre Bildung der Schiff'schen Base zu ermöglichen. Als Reaktionstemperatur wählt man hierbei bevorzugt einen Bereich zwischen 45 und 65°C. Nach einer gewissen Reaktionszeit, wie von einer bis vier Stunden, stellt man sodann den sauren pH-Bereich, wie zwischen 5,5 und 2, ein und führt den Ringschluß bei höherer Temperatur, wie bei einer Temperatur zwischen 60 und 90°C, durch.

Natürlich kann die Umsetzung der beiden Ausgangskomponenten auch sogleich im sauren pH-Bereich durchgeführt werden; jedoch ist die Ausbeute und Reinheit des Endproduktes in einer zweischrittigen Verfahrensweise etwas günstiger.

Es ist bei dem erfindungsgemäßen Verfahren nicht erforderlich, die Ausgangverbindungen (2-Hydroxy-cyclohexanon und m-Aminophenol) in reiner Form in den Reaktionsansatz einzubringen. Sie können in Form einer wäßrigen oder wäßrig-organischen Lösung (wobei das organische Lösemittel das erwähnte polare, mit Wasser mischbare organische Lösemittel ist) dem Reaktionsansatz zugeführt werden. Dies gilt insbesondere für 2-Hydroxy-cyclohexanon, das nur schwierig isolierbar in reiner Form erhältlich ist (s. hierzu die deutsche Offenlegungsschrift 2 155 561, Seiten 6 bis 12). Vorteilhaft kann deshalb im erfindungsgemäßen Verfahren das 2-Hydroxy-cyclohexanon in Form des Ansatzes, der aus dessen Synthese, wie beispielsweise der aus Hydrolyse eines 2-Halogeno-cyclohexanons, erhältlich ist, eingesetzt werden.

So läßt sich beispielsweise das 2-Hydroxy-cyclohexanon besonders vorteilhaft in das erfindungsgemäße Verfahren in Form einer wäßrigen oder wäßrig-organischen Lösung einsetzen, die bei der Synthese von 2-Brom-cyclohexanon und dessen nachfolgender Hydrolyse zum 2-Hydroxy-cyclohexanon gemäß dem Verfahren der am gleichen Tage eingereichten europäischen Patentanmeldung Nr.          (internes Aktenzeichen der Anmelderin: HOE 84/F 195) entsprechend der deutschen Prioritätsanmeldung P 34 31 415.6 vom 27. August 1984 erhältlich ist. Solch eine für das vorliegende erfindungsgemäße Verfahren wäßrige Ausgangslösung des 2-Hydroxy-cyclohexanons kann in der Weise erhalten werden, daß man Cyclohexanon in einem wäßrigen oder einem wäßrig-organischen Medium (wobei das organische Medium das oben erwähnte mit Wasser mischbare polare organische Lösemittel ist) im sauren pH-Bereich (von 0 bis 4) durch Einwirkung von einem Halogenatsalz, wie vorzugsweise einem Alkalichlorat, und Brom oder durch Einwirkung eines Halogenatsalzes und bei einer Temperatur zwischen bevorzugt 10 und 40°C bromiert (wobei man pro Mol Cyclohexanon mindestens 1/6 Mol Halogenatsalz und 1/2 Mol Brom bzw. mindestens 1/3 Mol Halogenatsalz und 1 Mol Bromwasserstoff einsetzt) und die anschließende Hydrolyse des gebildeten 2-Bromcyclohexanons ohne dessen Zwischenisolierung bei einer Temperatur zwischen 10 und 100°C und einem pH-Wert von oberhalb 7,5, vorzugsweise zwischen 8 und 12, durchführt. Nach erfolgter Hydrolyse kann diese Syntheselösung direkt - nach vorheriger Einstellung des erforderlichen pH-Wertes von 7,5 oder darunter - in das vorliegende erfindungsgemäße Verfahren eingesetzt werden; es ist hier bei ohne Belang, daß diese wäßrige Lösung aus der Synthese her Alkalihalogenide mit in das erfindungsgemäße Verfahren einbringt. Vielmehr ist sogar ein Anteil an einem Elektrolyten, wie einem Alkalihalogenid, günstig für eine Ausbeuteerhöhung.

Aus der nach erfolgter Synthesereaktion gemäß vorliegender Erfindung erhaltenen, in der Regel mittelstark sauren Lösung des 2-Hydroxy-5,6,7,8-tetrahydrocarbazols kann dieses in üblicher Weise, beispielsweise durch zusätzliche Zugabe einer starken Säure, isoliert und in erfindungsgemäßer Weise anschließend durch Überführung in ein Alkali- oder Erdalkalisalz gereinigt werden. Vorteilhafter ist es jedoch, das gebildete 2-Hydroxy-5,6,7,8-tetrahydrocarbazol aus der Synthesemischung durch Zusatz der entsprechenden Menge an einem Alkali- oder Erdalkalihydroxid, -oxid oder -carbonat als deren Alkali- oder Erdalkalisalz, wie beispielsweise Natrium-, Kalium-, Lithium- oder Calciumsalz, abzuscheiden und zu isolieren. Bevorzugt wird die Verbindung als Natriumsalz isoliert. Zur Abscheidung des 2-Hydroxy-5,6,7,8-tetrahydrocarbazols als Alkali- oder Erdalkalisalz wird im allgemeinen die Syntheselösung mit der wäßrigen Lösung oder Aufschlämmung des Alkali- oder Erdalkalihydroxids versetzt und auf eine Temperatur zwischen 15 und 25°C abgekühlt. Hierbei kristallisiert das gewünschte Alkali- oder Erdalkalisalz aus; es kann durch Filtration isoliert werden. Auch bei diesen Verfahrensmaßnahmen ist es vorteilhaft, unter einem inerten Schutzgas, wie beispielsweise unter Stickstoffatmosphäre, zu arbeiten.

Die Alkali- und Erdalkalisalze des 2-Hydroxy-5,6,7,8-tetrahydrocarbazols sind neu. Sie dienen erfindungsgemäß vorteilhaft zur Herstellung von reinem 2-Hydroxy-5,6,7,8-tetrahydrocarbazol und somit vorteilhaft zur Herstellung von 2-Hydroxycarbazol durch katalytische Dehydrierung. Das in der Dehydrierungsreaktion als Ausgangsverbindung dienende 2-Hydroxy-5,6,7,8,-tetrahydrocarbazol muß, wie bereits oben erwähnt, sehr rein sein; die neuen Alkali- und Erdalkalihydroxide des 2-Hydroxy-5,6,7,8-tetrahydrocarbazols sind vorteilhafte Zwischenprodukte für die Herstellung von 2-Hydroxy-carbazol und somit zur Synthese von reinen Farbstoffen, wie solchen in der Eisfarbenfärberei. Die erfindungsgemäßen Alkali- und Erdalkalisalze des 2-Hydroxy-5,6,7,8-tetrahydrocarbazols können aber auch direkt in eine Dehydrierungsreaktion, wie sie beispielsweise analog den bekannten Dehydrierungsreaktionen ausgeführt werden kann, eingesetzt werden; die erfindungsgemäß erhältlichen Salze besitzen hierfür die erforderliche Reinheit.

Aus dem isolierten Alkali- oder Erdalkalisalz des 2-Hydroxy-5,6,7,8-tetrahydrocarbazols kann die freie Verbindung in an und für sich üblicher Weise mittels einer starken wäßrigen Säure, wie beispielsweise Salzsäure oder auch im Falle der Alkalisalze Schwefelsäure, gegebenenfalls nach vorherigem Lösen in Wasser, freigesetzt werden. Das aus der sauren Lösung ausgefallene 2-Hydroxy-5,6,7,8-tetrahydrocarbazol wird abfiltriert, mit Wasser nachgewaschen und getrocknet.

Der Reinheitsgrad des erfindungsgemäß erhältlichen 2-Hydroxy-5,6,7,8-tetrahydrocarbazols entspricht dem eines gemäß dem Stand der Technik erhältlichen und durch Umkristallisation gereinigten Produktes; er ist ausreichend zur problemlosen Weiterverarbeitung dieses Produktes zum 2-Hydroxy-carbazol durch katalytische Dehydrierung und zur mehrmaligen Wiederverwendung des in der Dehydrierungsreaktion benutzten Katalysators.

Die nachfolgenden Beispiele dienen zur Erläuterung der Erfindung. Teile sind Gewichtsteile und Prozentangaben beziehen sich auf Gewichtsprozente, sofern nicht anders vermerkt. Die angegebenen Schmelzpunkte sind unkorrigiert. Die Reingehaltsbestimmungen erfolgten durch Hochdruck-Flüssigkeitschromatographie (HPLC-Analyse).

Beispiel 1

Eine Lösung von 57 Teilen 2-Hydroxy-cyclohexanon in 190 Teilen Wasser wird unter Stickstoff als Schutzgas mit 53 Teilen m-Aminophenol verrührt; der Ansatz wird bei einem pH-Wert von 7 und bei einer Temperatur von 50°C etwa 4 Stunden unter Rühren erhitzt. Sodann werden etwa 4 Teile einer wäßrigen 5n-Schwefelsäure zugegeben, der Ansatz auf 70 bis 80°C erwärmt und unter weiterem Rühren 4 Stunden bei dieser Temperatur beibehalten. Anschließend gibt man 68 Teile einer etwa 33%igen wäßrigen Natronlauge hinzu, rührt den Ansatz ohne zu heizen weiter, bis sich das Natriumsalz kristallin abgeschieden hat, und filtriert dieses nach Abkühlen der Lösung auf Raumtemperatur unter Stickstoffatmosphäre ab, wäscht es mit einer gesättigten wäßrigen Natriumchloridlösung und saugt es gut trocken.

Das Natriumsalz des 2-Hydroxy-5,6,7,8-tetrahydrocarbazols wird anschließend in eine Mischung aus 400 Teilen Wasser und 43 Teilen einer etwa 30%igen wäßrigen Salzsäure eingerührt. Es wird noch einige Stunden nachgerührt und das ausgeschiedene 2-Hydroxy-5,6,7,8-tetrahydracarbazol abgesaugt, mit Wasser gewaschen und unter reduziertem Druck bei 60°C getrocknet. Es werden 42 Teile 2-Hydroxy-5,6,7,8-tetrahydrocarbazol mit einem Schmelzpunkt von 153 bis 156°C und mit einem Reingehalt von 86 % erhalten.

Beispiel 2

Man verfährt gemäß der Verfahrensweise des Beispieles 1, setzt jedoch noch zusätzlich 145 Teile Methanol zur wäßrigen Lösung des 2-Hydroxy-cyclohexanons hinzu. Nach Aufarbeitung gemäß den Angaben des Beispieles 1 erhält man 40 Teile 2-Hydroxy-5,6,7,8-tetrahydrocarbazol mit einem Schmelzpunkt von 161 bis 162°C und einem Reingehalt von 93 % d.Th..

Beispiel 3

2300 Teile einer wäßrigen, neutralen Lösung von 460 Teilen
2-Hydroxy-cyclohexanon, die zusätzlich noch etwa 650 Teile
Natriumchlorid enthält, werden unter Stickstoffatmosphäre
zusammen mit 410 Teilen m-Aminophenol und etwa 900 Teilen
Methanol unter Rühren auf 50°C erhitzt; der Ansatz wird bei
dieser Temperatur noch 3 Stunden unter weiterem Rühren
gehalten.

Anschließend gibt man langsam etwa 40 Teile einer wäßrigen
5n-Schwefelsäure hinzu, erhitzt den Ansatz auf 70 bis 80°C
und rührt ihn innerhalb dieses Temperaturbereiches noch
4 Stunden weiter.

Sodann gibt man 400 Teile einer etwa 33%igen wäßrigen
Natronlauge hinzu, läßt unter Rühren das Reaktionsgemisch
auf Raumtemperatur abkühlen, saugt das gebildete Natriumsalz des 2-Hydroxy-5,6,7,8-tetrahydrocarbazols ab, wäscht
es mit gesättigter wäßriger Natriumchloridlösung, trägt
dieses Salz sodann in eine verdünnte wäßrige Schwefelsäure
ein und isoliert daraus das abgeschiedene freie,2-Hydroxy-
5,6,7,8-tetrahydrocarbazol, beispielsweise durch Filtration, wäscht das Produkt mit Wasser nach und trocknet es
unter reduziertem Druck bei 60°C.

Ausbeute: 453 Teile 2-Hydroxy-5,6,7,8-tetrahydrocarbazol
          (Smp.: 159-161°C; 92%ig).

Beispiel 4

a) 103,8 Teile Natriumchlorat werden in 555 Teilen einer
   etwa 6%igen wäßrigen Salzsäure gelöst, und 435 Teile
   Cyclohexanon werden zugesetzt. Unter Rühren gibt man
   innerhalb von etwa 60 Minuten bei einer Temperatur von
   20 bis 25°C insgesamt 390,6 Teile Brom hinzu (die
   Reaktionstemperatur wird durch leichte Außenkühlung gehalten). Man rührt noch kurze Zeit nach und läßt sodann
   langsam 648 Teile einer 33%igen wäßrigen Natronlauge
   zulaufen, wobei man die Temperatur des Reaktionsgemisches auf bis zu 50°C ansteigen läßt. Man rührt noch zwei Stunden nach und stellt den Ansatz mit wenig verdünnter wäßriger Salzsäure auf einen pH-Wert von 7 ein. Es werden etwa 2150 Teile einer wäßrigen, neutralen Lösung von 460 Teilen 2-Hydroxy-cyclohexanon mit etwa 510 Teilen Natriumbromid und etwa 150 Teilen Natriumchlorid erhalten.

b) Die unter a) erhaltene wäßrige Lösung des 2-Hydroxycyclohexanons wird unter Stickstoffatmosphäre nach Zusatz von 410 Teilen m-Aminophenol und etwa 950 Teilen Methanol während etwa 4 Stunden unter Rühren bei 50°C erhitzt. Anschließend gibt man etwa 40 Teile einer wäßrigen 5n-Schwefelsäure hinzu und führt die Umsetzung unter Rühren noch etwa 4 Stunden bei einer Temperatur zwischen 70 und 80°C weiter. Man läßt sodann unter Rühren 480 Teile einer etwa 33%igen wäßrigen Natronlauge zulaufen und rührt das Ganze noch bis zur Abkühlung auf Raumtemperatur weiter. Das ausgeschiedene Natriumsalz des 2-Hydroxy-5,6,7,8-tetrahydrocarbazols wird abgesaugt, mit gesättigter wäßriger Natriumchloridlösung gewaschen und gut trocken gesaugt.

Das Natriumsalz kann durch Eintragen in eine verdünnte wäßrige Salzsäure, beispielsweise analog den Angaben des Beispieles 1, in das freie 2-Hydroxy-5,6,7,8-tetrahydrocarbazol überführt werden. Nach dessen Isolierung, Waschen mit Wasser und Trocknung bei 60°C unter reduziertem Druck erhält man 430 Teile 2-Hydroxy-5,6,7,8-tetrahydrocarbazol (Smp.: 161-163°C; 95%ig).

Beispiel 5

Zur Herstellung von 2-Hydroxy-5,6,7,8-tetrahydrocarbazol verfährt man gemäß der in Beispiel 4 angegebenen Verfahrensweise, verwendet jedoch anstelle von 950 Teilen Methanol 1600 Teile Methanol und setzt zur Salzbildung anstelle von 480 Teilen einer 33%igen Natronlauge 660 Teile einer 33%igen wäßrigen Kalilauge hinzu.

Nach Zugabe der Kalilauge rührt man bis zum Abkühlen des Ansatzes und saugt das abgeschiedene Kaliumsalz des 2-Hydroxy-5,6,7,8-tetrahydrocarbazols ab. Die Überführung des Kaliumsalzes in die freie Hydroxyverbindung erfolgt wie im Beispiel 4 angegeben.

Ausbeute: 360 Teile 2-Hydroxy-5,6,7,8-tetrahydrocarbazol mit einem Schmelzpunkt von 158-161°C und einem Reingehalt von 90%.

Beispiel 6

Man verfährt zur Herstellung der 2-Hydroxy-5,6,7,8-tetrahydrocarbazols und dessen Alkalisalz gemäß der Verfahrensweise des Beispieles 4, setzt jedoch anstelle von 950 Teilen Methanol 1600 Teile Methanol ein und verwendet zur Salzbildung anstelle von 480 Teilen 33%iger Natronlauge 670 Teile einer etwa 15%igen wäßrigen Lithiumhydroxidlösung. Man läßt den Ansatz unter Rühren abkühlen und saugt das abgeschiedene Lithiumsalz des 2-Hydroxy-5,6,7,8-tetrahydrocarbazols ab, wäscht es mit gesättigter Natriumchloridlösung nach und trocknet es unter reduziertem Druck bei 60°C.

Die Überführung des Lithiumsalzes in das 2-Hydroxy-5,6,7,8-tetrahydrocarbazols erfolgt durch Zugabe in eine wäßrige Säure, wie beispielsweise im Beispiel 1 für das Natriumsalz beschrieben.

Beispiel 7

Zur Herstellung des Calciumsalzes des 2-Hydroxy-5,6,7,8-tetrahydrocarbazols verfährt man gemäß der Verfahrensweise des Beispieles 4, setzt jedoch anstelle von 950 Teilen Methanol 1600 Teile Methanol hinzu und führt die Salzbildung anstelle von 480 Teilen 33%iger Natronlauge mit einer Aufschlämmung von 200 Teilen Calciumhydroxid in 400 Teilen Wasser durch. Man rührt den heißen Ansatz noch weiter, bis er auf etwa 25°C abgekühlt ist, saugt das abgeschiedene Calciumsalz des 2-Hydroxy-5,6,7,8-tetra- hydrocarbazols ab, wäscht es mit gesättigter Natriumchloridlösung nach und trocknet es unter reduziertem Druck bei 60°C.

Aus dem Calciumsalz kann mittels einer wäßrigen Säure das freie 2-Hydroxy-5,6,7,8-tetrahydrocarbazol gewonnen werden, beispielsweise analog den Angaben des Beispieles 1 aus dem Natriumsalz.

Beispiel 8

Zur Herstellung des Magnesiumsalzes des 2-Hydroxy-5,6,7,8-tetrahydrocarbazols verfährt man gemäß der Verfahrensweise des Beispieles 4, setzt jedoch anstelle der 950 Teile Methanol 1600 Teile Methanol ein und verwendet zur Salzbildung anstelle der Natronlauge 243 Teile einer Suspension von Magnesiumhydroxid in 480 Teilen Wasser. Der heiße Ansatz wird nach Neutralisation bis zur Abkühlung auf 25°C weitergerührt, das abgeschiedene Magnesiumsalz des 2-Hydroxy-5,6,7,8-tetrahydrocarbazols abfiltriert, mit gesättigter Natriumchloridlösung gewaschen und getrocknet.

Aus dem Magnesiumsalz kann mittels einer wäßrigen Säure das freie 2-Hydroxy-5,6,7,8-tetrahydrocarbazol gewonnen werden, beispielsweise analog den Angaben des Beispieles 1 wie aus dem Natriumsalz.

Beispiel 9

Man verfährt gemäß der Verfahrensweise des Beispieles 9, verwendet jedoch anstelle der angegebenen wäßrigen Magnesiumhydroxid-Suspension eine Suspension von 210 Teilen Magnesiumcarbonat in 800 Teilen Wasser.
Das gebildete Magnesiumsalz des 2-Hydroxy-5,6,7,8-tetrahydrocarbazols wird isoliert und kann gemäß den Angaben des Beispieles 9 in das freie 2-Hydroxy-5,6,7,8-tetrahydrocarbazol übergeführt werden.

<u>Verwendungsbeispiel</u> für die Salze des 2-Hydroxy-5,6,7,8-
tetrahydrocarbazols:


40 Teile eines wasserfeuchten Natriumsalzes des 2-Hydroxy-
5,6,7,8-tetrahydrocarbazols, wie es beispielsweise nach
Beispiel 4 hergestellt wurde, mit einem Trockengehalt von
etwa 60% werden in 130 Teilen 2-Ethyl-hexan-1-ol gelöst
und in Gegenwart von 20 Teilen eines üblichen Palladium-
Aktivkohle Katalysators (mit einem Palladiumgehalt von
5 %) bei 150°C dehydriert; hierbei werden gleichzeitig
Wasser und geringe Anteile an Methanol abdestilliert. Die
Reaktionszeit beträgt etwa fünf Stunden. Anschließend wird
der Katalysator aus dem heißen Ansatz abfiltriert, das
Filtrat auf die Hälfte seines Volumens durch Abdestillation reduziert und auf Raumtemperatur abgekühlt. Das ausgeschiedene 2-Hydroxy-carbazol wird abgesaugt und getrocknet.
Ausbeute: 18 Teile entspr. 86 % d.Th.; Smp.: 265-267°C.


Der Katalysator, das Lösemittel enthaltende Filtrat und
das abdestillierte Lösemittel können mehrfach ohne Reinigung in nachfolgende Dehydrierungseinsätze eingesetzt werden, ohne daß Ausbeute und Reinheit des 2-Hydroxy-carba-
zols absinken.


<u>Vergleichsbeispiel</u>
Gemäß den Angaben in Chem. Ber. <u>92</u>, 2395 (1959) wurden
5,5 Teile m-Aminophenol und 5,7 Teile 2-Hydroxy-cyclohexanon in einem Ölbad mit einer Badtemperatur von 125 bis
130°C 20 Minuten lang erhitzt; sodann wurden einige
Tropfen einer konzentrierten wäßrigen Salzsäure hinzugefügt und die Badtemperatur noch etwa 30 Minuten bei
125 bis 130°C gehalten. Die sodann auf 60°C abgekühlte
Schmelze wurde in 50 Teilen Methanol gelöst; bei einer
Temperatur von etwa 60°C wurde bis zur beginnenden Trübe
der methanolischen Lösung Wasser hinzugefügt. Nach anschließendem Erkalten wurden 7,1 Teile eines abgeschiedenen dunkelbraunen Produktes isoliert, das unter reduziertem Druck bei 60°C getrocknet wurde. Dieses Produkt begann bereits bei 100°C zu schmelzen und war bei 147°C vollständig geschmolzen. Der Reingehalt dieses Produktes an 2-Hydroxy-5,6,7,8-tetrahydrocarbazol lag bei 65 %.

Patentansprüche:

1. Verfahren zur Herstellung von 2-Hydroxy-5,6,7,8-tetrahydrocarbazol und dessen Alkali- und Erdalkalisalze durch Umsetzung von 2-Hydroxy-cyclohexanon mit m-Aminophenol, dadurch gekennzeichnet, daß man die Umsetzung in wäßrigem Medium oder wäßrig-organischem Medium, wobei das organische Lösemittel ein mit Wasser mischbares, polares organisches Lösemittel ist, bei einer Temperatur zwischen 20 und 100°C und einem pH-Wert zwischen 7,5 und 1 durchführt, das gebildete 2-Hydroxy-5,6,7,8-tetrahydrocarbazol aus der Reaktionsmischung als Alkali- oder Erdalkalisalz abscheidet und dieses Salz gegebenenfalls mittels einer Mineralsäure in das freie 2-Hydroxy-5,6,7,8-tetrahydrocarbazol überführt.

2. Verfahren nach Anspruch 1, dadurch gekennzeichnet, daß man die Umsetzung des 2-Hydroxy-cyclohexanons mit dem m-Aminophenol in einem wäßrigen, ein mit Wasser mischbares, polares organisches Lösemittel enthaltenden Medium durchführt.

3. Verfahren nach Anspruch 2, dadurch gekennzeichnet, daß das mit Wasser mischbare, polare organische Lösemittel ein Alkanol von 1 bis 5 C-Atomen ist.

4. Verfahren nach Anspruch 3, dadurch gekennzeichnet, daß das organische Lösemittel Methanol ist.

5. Verfahren nach einem der Ansprüche 1 bis 4, dadurch gekennzeichnet, daß man das 2-Hydroxy-cyclohexanon in Form seiner wäßrigen oder wäßrig-organischen Lösung in die Umsetzung mit dem m-Aminophenol einsetzt.

6. Verfahren nach Anspruch 5, dadurch gekennzeichnet, daß man zunächst Cyclohexanon in einem wäßrigen oder wäßrig-organischem Medium (wobei das organische Lösemittel ein mit Wasser mischbares, polares Lösemittel
ist) bei einer Temperatur zwischen 10 und 50°C im sauren pH-Bereich durch Einwirkung eines Bromierungsagenz,
bestehend aus mindestens 1/6 Mol Halogenatsalz und
1/2 Mol Brom oder aus mindestens 1/3 Mol Halogenatsalz
und 1 Mol Bromwasserstoffsäure, bezogen auf 1 Mol
Cyclohexanon, bromiert und das gebildete 2-Brom-cyclohexanon ohne dessen Zwischenisolierung bei einer Temperatur von 10 bis 100°C und einem pH-Wert von oberhalb
7,5 zum 2-Hydroxy-cyclohexanon hydrolisiert, die so
erhaltene wäßrige oder wäßrig-organische Lösung des
2-Hydroxy-cyclohexanons auf einen pH-Wert von 7,5 oder
darunter einstellt, diese Lösung mit m-Aminophenol,
gegebenenfalls in Wasser oder einem mit Wasser mischbaren organischen polaren Lösemittel oder einer
Mischung derselben gelöst oder suspendiert, versetzt
und die Umsetzung der beiden Komponenten gemäß der Verfahrensweise des Anspruchs 1 durchführt.

7. Verfahren nach einem der Ansprüche 1 bis 6, dadurch
gekennzeichnet, daß man unter Ausschluß von Luftsauerstoff arbeitet.

8. Verfahren nach einem der Ansprüche 1 bis 7, dadurch
gekennzeichnet, daß man das Natriumsalz des 2-Hydroxy-
5,6,7,8-tetrahydrocarbazols herstellt und isoliert.

9. Ein Alkali- oder Erdalkalisalz des 2-Hydroxy-5,6,7,8-
tetrahydrocarbazols.

10. Das Natriumsalz des 2-Hydroxy-5,6,7,8-tetrahydro-
carbazols.

11. Das Kaliumsalz des 2-Hydroxy-5,6,7,8-tetrahydrocarbazols.

12. Verwendung eines Alkali- oder Erdalkalisalzes des 2-Hydroxy-5,6,7,8-tetrahydrocarbazols zur Reindarstellung von 2-Hydroxy-5,6,7,8-tetrahydrocarbazol.

13. Verwendung eines Alkali- oder Erdalkalisalzes des 2-Hydroxy-5,6,7,8-tetrahydrocarbazols zur Synthese von 2-Hydroxy-carbazol durch katalytische Dehydrierung.